# EUROPEAN PATENT APPLICATION

(11) **EP 3 919 082 A1**
(43) Date of publication of application: **08.12.2021**
(21) Application number: 20178179.6
(22) Date of filing: 04.06.2020
(51) Int. Cl.: A61K 51/04, A61K 101/02, A61P 35/00

(54) **PSMA TARGETING UREA-BASED LIGANDS FOR PROSTATE CANCER RADIOTHERAPY AND IMAGING**

(71) Applicant: Rigshospitalet, 2100 Copenhagen Ø (DK); University of Copenhagen, 2100 Copenhagen Ø (DK); Technical University of Denmark, 4000 Roskilde (DK)
(72) Inventor: KJÆR, Andreas, 2000 Frb (DK); HERTH, Matthias Manfred, 21121 Malmö (SE); JENSEN, Andreas Ingemann, 2830 Virum (DK)
(74) Representative: Budde Schou A/S

(57) **Abstract**

The present invention provides novel PSMA targeting urea-based ligands that bind to prostate-specific membrane antigen (PSMA) which is expressed 8-to-12-fold higher in prostate cancer cells when compared to healthy tissue. The PSMA targeting urea-based ligands comprise a chelating agent that may comprise a metal and a halogen radioisotope of fluorine, iodine, bromine or astatine. The invention further relates to a method for providing the PSMA targeting urea-based ligands of the invention, to precursors of the PSMA targeting urea-based ligands and to the PSMA targeting urea-based ligands use in radiotherapy, imaging and theranostics.

## Description

### Field of the Invention

The present invention relates to urea-based ligands specifically targeting a prostate-specific membrane antigen and their use in radiotherapy and imaging.

### Background of the invention

Prostate cancer (PC) is one of the most commonly diagnosed diseases in men.¹ Moreover, a significant amount of people suffering from PC will develop bone metastases, which results in a 1-year survival rate of only 40 %. Some of the patients are non-responders to conventional hormonal therapy, developing what is known as castration-resistant prostate cancer (CRPC).^{2,3} Limited options are available for patients suffering from CRPC, for which reason the development of highly specific and potent radiopharmaceuticals is of the utmost interest. Targeting prostate-specific membrane antigen (PSMA) which is expressed 8-to-12-fold higher in PC cells when compared to healthy tissue,⁴ offers the possibility for bio-specific imaging and treatment of PC. Nevertheless, some of the developed PSMA-targeting radiopharmaceuticals have significantly unpleasant side effects, like renal toxicity and salivary gland build-up.

The use of small molecular weight ligands for selectively targeting PSMA stands to reason, as it fulfils all the requirements for radiotherapy. Small molecules exhibit the best pharmacokinetic properties such as short half-life in the bloodstream and fast clearance. In contrast, big biological entities such as antibodies have much longer circulation times and a very slow clearance profile. Long circulation time and a slow clearance profile makes a compound less suitable for radiotherapy, since the extended presence of the noxious radioactive payload in the bloodstream translates into unwanted damage in non-target tissue. In the small-molecule class, urea-based ligands have been shown to exhibit high affinities for PSMA, with very low nonspecific binding, high tumour accumulation over time and fast clearance.

The current state-of-the-art in PSMA targeted radiotherapy is based on ¹⁷⁷Lu-PSMA-617, which has shown good molecular response in clinical evaluations, clearing a noticeable amount of metastases and significantly reducing PSA concentrations to normal levels (below 4.0 ng/mL).⁵ Nevertheless, a notable 30% of patients do not respond to β⁻-emitter based therapy such as ¹⁷⁷Lu-PSMA-617, for which reason an alternative strategy is to use cytotoxic alpha-emitters instead.

Efforts have been made in targeting PSMA for therapy employing alpha-emitters. For example, ²²⁵Ac has been used but it is not an ideal radionuclide for therapy as it decays through a chain that includes four α-active daughter radionuclides. Combined with a decay half-life of 10 days, this means that extensive damage can be caused to healthy tissue once the radionuclides are expelled from the chelator. Our approach is based on the use of Astatine-211 (²¹¹At). Astatine-211 is one of the most appealing radionuclides for alpha-radiotherapy. Its short half-life of 7.2 hours is in accordance with the pharmacokinetics of small-molecule urea-based PSMA ligands. Moreover, its decay pathways do not include any long-lived alpha-emitting daughter that could be expelled into the bloodstream from the binding site. This significantly reduces any unwanted cytotoxicity to the patient.

Radiohalogenated PSMA-targeting pharmaceuticals have been developed and have shown good, specific tumour uptake, but these compounds were marred by renal and salivary gland build-up, when no blocking agents were administered.⁶

Other radiopharmaceuticals developed (WO2019157037A1) were urea-based and DOTA containing, but also contained an aliphatic chain as well as a secondary amine as key features. Secondary amides are prone to hydrolysis *in vivo.* The presence of a cyclohexyl group as a linker between the urea and the DOTA chelator has shown to favour internalisation and thus, tumour accumulation.^{5,7}

The PSMA targeting urea-based ligands for prostate cancer radiotherapy and imaging disclosed herein are based upon peptide bonds. Thus, they do not bear any secondary amides as the radiohalogen bearing moiety and are more stable under physiological conditions. The ease of synthesis of amide bonds through chemically modified amino acids makes these PSMA targeting urea-based ligands for prostate cancer radiotherapy and imaging easy to manufacture in an automated, resin-bound or solid-phase process if needed to scale up for routine clinical use.

### Summary of the invention

The present invention provides novel PSMA targeting urea-based ligands and the use of these compounds in radiotherapy and imaging is disclosed.

The PSMA targeting urea-based ligands of the present invention have the following general formula (I): 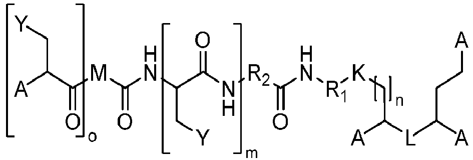 wherein:
A is independently carboxylic acid, sulphonic acid, phosponic acid, tetrazole or isoxazole;
L is selected from the group consisting of urea, thiourea, -NH-(C=O)-O-, -O-(C=O)-NH- or -CH₂-(C=O)-CH₂-
K is selected from the group consisting of -(C=O)-NH-, -CH₂-NH-(C=O)- or 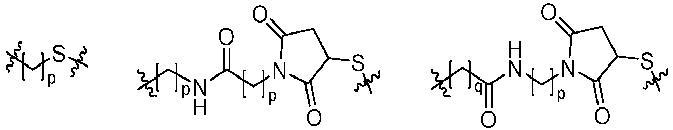 wherein
p is independently an integer selected from the group consisting of 1, 2, 3, 4, 5 and 6 ;
q is an integer selected from the group consisting of 0, 1, 2, 3, 4, 5 and 6 ;
Y is selected from the group consisting of: 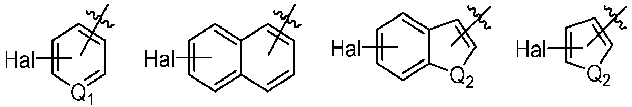 wherein
Q₁ is -C-R₃ or N, wherein R₃ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
M is a chelating agent, that can comprise a metal
n is an integer selected from the group consisting of 1, 2, 3, 4, 5 and 6 ;
m is an integer selected from the group consisting of 0 and 1;
o is an integer selected from the group consisting of 0 and 1;
R₁ is -CH-CH₂-Z or -CH-CH₂-Y;
wherein Z is selected from the group consisting of: 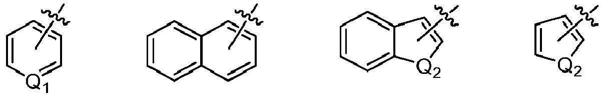 and Y is selected from the group consisting of: 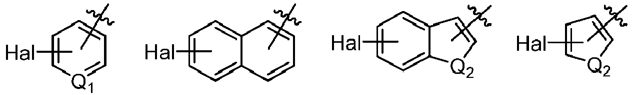 wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
R₂ is -CH-CH₂-Y or -CH₂-X-;
wherein X is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 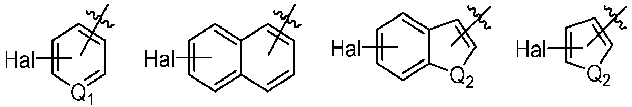
wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
and wherein formula (l) comprises at least one radioisotope selected from a radioisotope of fluorine, a radioisotope of iodine, a radioisotope of bromine or a radioisotope of astatine;
and pharmaceutically acceptable salts thereof.

The suitability of the compounds of Formula (l) in radiotherapy and imaging is shown.

### Brief description of the drawings

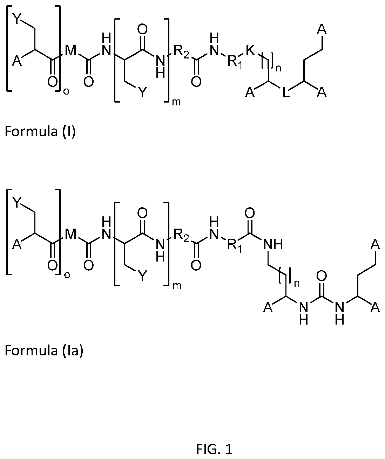

Figure 1 shows the structural formula (I) and (Ia) of the compounds of the present invention.

### Detailed description of the invention

The PSMA targeting urea-based ligands of the present invention are suitable for use as radiopharmaceuticals, either as imaging agents or for the treatment of prostate cancer, or as theranostic agents.

The PSMA targeting urea-based ligands of the present invention takes advantage of a urea-based binding motif (((S)-5-amino-1-carboxypentyl)carbamoyl)-L-glutamic acid). This motif specifically interacts with the PSMA antigen binding pocket. It contains an urea that forms a coordination complex with a Zn⁺² atom, which is crucial for the binding. Moreover, carboxylic acids also interact with the residues in the vicinity of the binding site, making this scaffold very convenient for PSMA-specific targeting.

The pharmacokinetics of the presently disclosed compounds outperform the already existing astatine radionuclide based approaches.

The PSMA targeting urea-based ligands of the present invention have the following general formula (I): 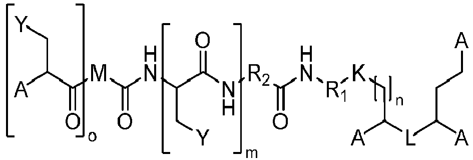 wherein:
A is independently carboxylic acid, sulphonic acid, phosponic acid, tetrazole or isoxazole;
L is selected from the group consisting of urea, thiourea, -NH-(C=O)-O-, -O-(C=O)-NH- or -CH₂-(C=O)-CH₂-;
K is selected from the group consisting of -(C=O)-NH-, -CH₂-NH-(C=O)- or 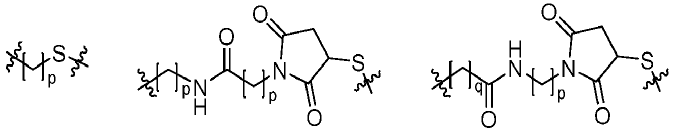
wherein
p is independently an integer selected from the group consisting of 1, 2, 3, 4, 5 and 6 ;
q is an integer selected from the group consisting of 0, 1, 2, 3, 4, 5 and 6 ;
Y is selected from the group consisting of: 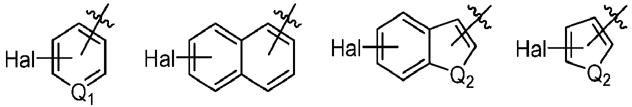
wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
M is a chelating agent, that can comprise a metal
n is an integer selected from the group consisting of 1, 2, 3, 4, 5 and 6 ;
m is an integer selected from the group consisting of 0 and 1;
o is an integer selected from the group consisting of 0 and 1;
R₁ is -CH-CH₂-Z or -CH-CH₂-Y;
wherein Z is selected from the group consisting of: 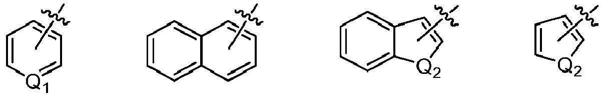
and Y is selected from the group consisting of: 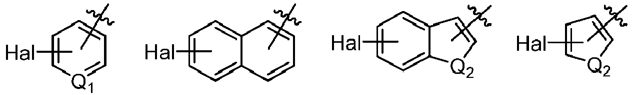
wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
R₂ is -CH-CH₂-Y or -CH₂-X-;
wherein X is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 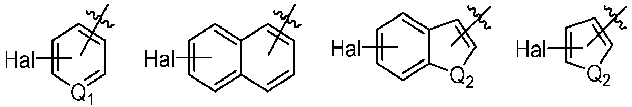 wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
and wherein formula (l) comprises at least one radioisotope selected from a radioisotope of fluorine, a radioisotope of iodine, a radioisotope of bromine or a radioisotope of astatine;
and pharmaceutically acceptable salts thereof.

In a particular embodiment, the PSMA targeting ligand of formula (I) is selected from the group of compounds of formula (Ia): 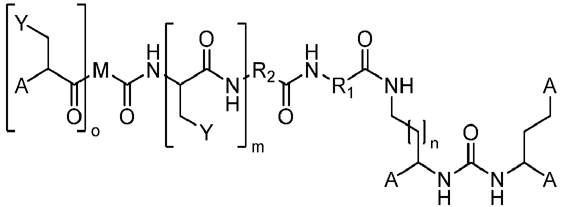 wherein:
A is independently carboxylic acid, sulphonic acid, phosponic acid, tetrazole or isoxazole;
n is an integer selected from the group consisting of 1, 2, 3 and 4 ;
m is an integer selected from the group consisting of 0 and 1;
o is an integer selected from the group consisting of 0 and 1;
Y is selected from the group consisting of: 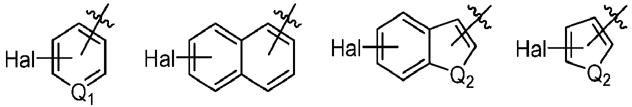 wherein
Q₁ is -C-R³ or N, wherein R³ is H or C₁-C₅ alkyl;
Q₂ is O, S or NH;
Hal is selected from the group consisting of a radioisotope of fluorine, a radioisotope of iodine,
a radioisotope of bromine or a radioisotope of astatine;
M is a chelating agent that can comprise a metal
R₁ is -CH-CH₂-Z or -CH-CH₂-Y;
wherein Z is selected from the group consisting of: 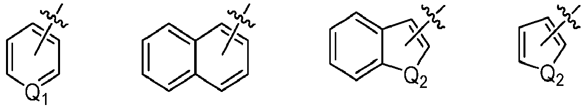
and Y is selected from the group consisting of: 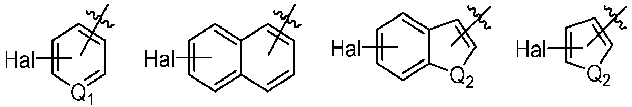 wherein
Q₁ is -C-R³ or N, wherein R³ is H or C₁-C₅ alkyl;
Q₂ is O, S or NH;
Hal (halogen) is selected from the group consisting of a radioisotope of fluorine, a radioisotope iodine, of a radioisotope of bromine or a radioisotope of astatine;
R₂ is -CH-CH₂-Y or -CH₂-X-;
wherein X is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 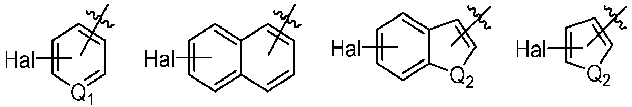 wherein
Q₁ is -C-R³ or N, wherein R³ is H or C₁-C₅ alkyl;
Q₂ is O, S or NH;
Hal is selected from the group consisting of a radioisotope of fluorine, a radioisotope of iodine, a radioisotope of bromine or a radioisotope of astatine;
and wherein formula (l) comprises at least one radioisotope selected from a radioisotope of fluorine, a radioisotope of iodine, a radioisotope of bromine or a radioisotope of astatine;
and pharmaceutically acceptable salts thereof.

The chelating agent may be selected from one of the following chelators:
1,4,7,10-tetraazacyclododecane-N,N',N',N"-tetraacetic acid (DOTA),
N,N'-bis(2-hydroxy-5-(carboxyethyl)benzyl)ethylenediamine N,N'-diacetic acid (HBED-CC), 14,7-triazacyclononane-1,4,7-triacetic acid (NOTA),
2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1-yl)pentanedioic acid (DOTAGA),
14,7-triazacyclononane phosphinic acid (TRAP),
14,7-triazacyclononane-1-methyl(2-carboxyethyl)phosphinic acid-4,7-bis(methyl(2-hydroxymethyl)phosphinic acid (NOPO),
3,6,9,15-tetraazabicyclo9.3.1.pentadeca-1 (15),11,13-triene-3,6,9- triacetic acid (PCTA),
N'-(5-acetyl (hydroxy)aminopentyl-N-(5-(4-(5- aminopentyl)(hydroxy)amino-4-oxobutanoyl)amino)pentyl-N- hydroxysuccinamide (DFO),
diethylenetriaminepentaacetic acid (DTPA),
trans-cyclohexyl-diethylenetriaminepentaacetic acid (CHX-DTPA),
1-oxa-4,7,10-triazacyclododecane-4,7,10-triacetic acid (OXO-Do3A),
p-isothiocyanatobenzyl-DTPA (SCN-BZ-DTPA),
1-(p-isothiocyanatobenzyl)-3-methyl-DTPA (1B3M),
2-(p-isothiocyanatobenzyl)-4-methyl-DTPA (1M3B),
1-(2)-methyl-4-isocyanatobenzyl-DTPA (MX-DTPA), that can comprise a metal; and pharmaceutically acceptable salts thereof.

In some embodiments, the chelating agent comprises a metal. In a preferred embodiment, the chelating agent comprises a metal selected from the group consisting of Y, Lu, Tc, Zr, In, Sm, Re, Cu, Pb, Ac, Bi, Al, Ga, Ho and Sc.

The radionuclide (Hal) maybe present in the R₁, R₂ and Y groups in Formula (I). The radionuclide is a radionuclide selected from the halogen group. This group comprises radionuclides of Fluorine (F), Chlorine (CI), Bromine (Br), Iodine (I) and Astatine (At).

In a preferred embodiment, the radionuclide is selected from a radioisotope of iodine, a radioisotope of bromine or a radioisotope of astatine.

In another preferred embodiment, the radionuclide (Hal) is selected from the group consisting of ¹²⁵I, ¹²³I, ¹³¹I, ¹²⁴I, ²¹¹At, ⁷⁷Br and ^{80m}Br.

In a particularly preferred embodiment, the radionuclide (Hal) is ^{123/124/125/131}I or ²¹¹At.

In preferred embodiments, the PSMA targeting ligand according to Formula (I), is one of the following seven compounds:

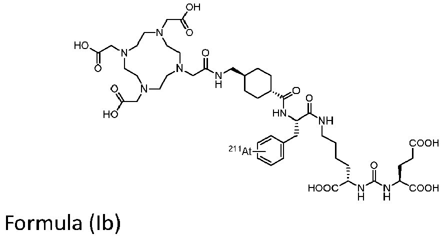

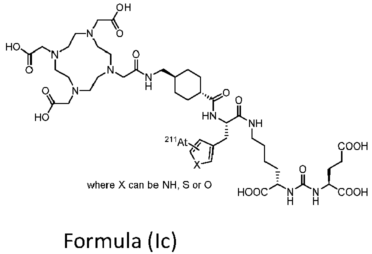

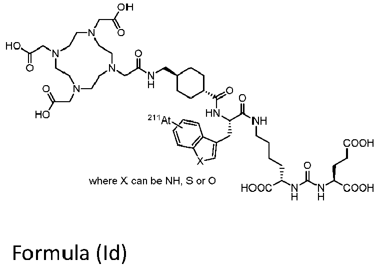

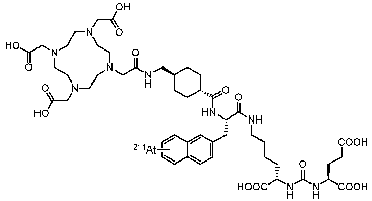

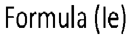

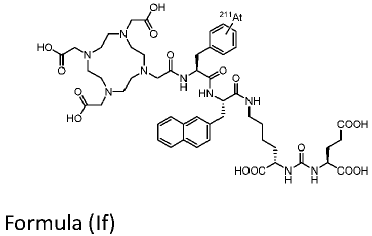

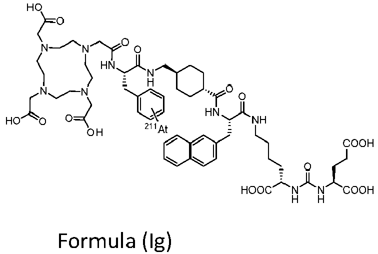

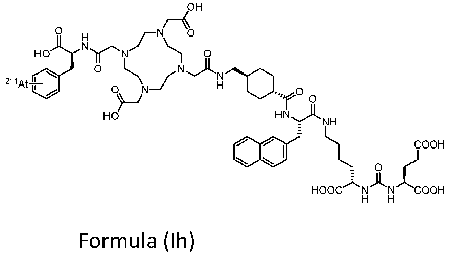

The PSMA targeting ligands according to formula (I) may be provided by suitable methods known in the art.

In one aspect, the present invention relates to a method for providing the PSMA targeting ligands according to Formula (I) comprising the steps of:
- Synthesis of a PSMA binding motif (BM)
- Coupling of linkers to BM
- Coupling of BM-linker to a chelator
- Labeling the BM-linker-chelator with a halogen radionuclide

In one embodiment, the PSMA binding motif is Lys-urea-Glu (LUG).

In one embodiment, the chelator is selected from:
1,4,7,10-tetraazacyclododecane-N,N',N',N"-tetraacetic acid (DOTA),
N,N'-bis(2-hydroxy-5-(carboxyethyl)benzyl)ethylenediamine N,N'-diacetic acid (HBED-CC), 14,7-triazacyclononane-1,4,7-triacetic acid (NOTA),
2-(4.7-bis(carboxymethyl)-1,4,7-triazonan-1-yl)pentanedioic acid (NODAGA),
2-(4,7,10-tris(carboxymethyl)-1,4,7,10-tetraazacyclododecan-1- yl)pentanedioic acid (DOTAGA),
14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO),
3,6,9,15-tetraazabicyclo9.3.1.pentadeca-1 (15),11,13-triene-3,6,9-triacetic acid (PCTA),
N'-(5-acetyl (hydroxy)aminopentyl-N-(5-(4-(5- aminopentyl)(hydroxy)amino-4-oxobutanoyl)amino)pentyl-N- hydroxysuccinamide (DFO),
diethylenetriaminepentaacetic acid (DTPA),
trans-cyclohexyl-diethylenetriaminepentaacetic acid (CHX-DTPA),
1-oxa-4, 7, 10-triazacyclododecane-4, 7, 10-triacetic acid (OXO-Do3A),
p-isothiocyanatobenzyl-DTPA (SCN-BZ-DTPA),
1-(p-isothiocyanatobenzyl)-3-methyl-DTPA (1B3M),
2-(p-isothiocyanatobenzyl)-4-methyl-DTPA (1M3B), and
1-(2)-methyl-4-isocyanatobenzyl-DTPA (MX-DTPA)

In one embodiment, the halogen radionuclide is selected from a radioisotope of iodine, bromine or astatine.

In a preferred embodiment, the halogen radionuclide is one of the following radionuclides: ¹²⁵I, ¹²³I, ¹³¹I, ¹²⁴I, ²¹¹At, ⁷⁷Br and ^{80m}Br.

The present invention also provides (Me)₃Sn precursors that can be used to provide the PSMA targeting ligand according to Formula (I).

These precursors include (Me)₃Sn precursors with and without chelators and have the following structures: 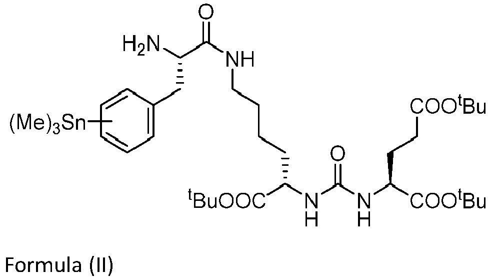 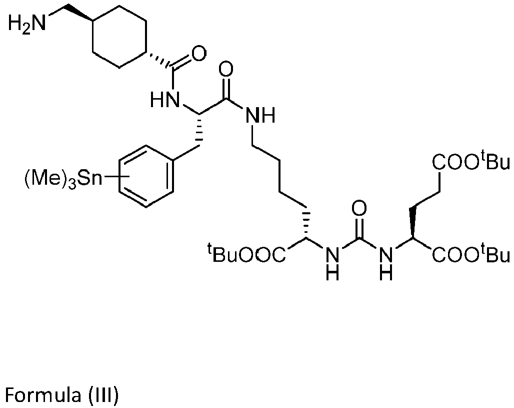 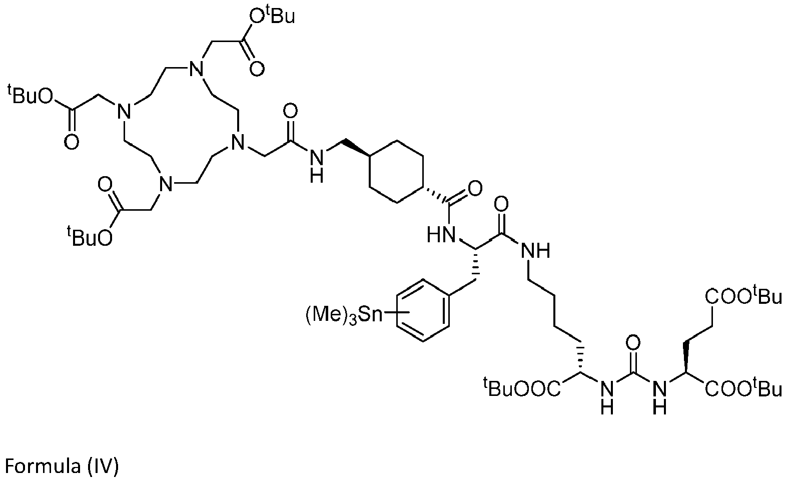 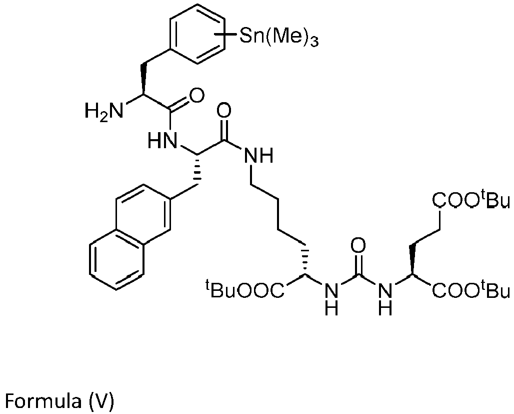 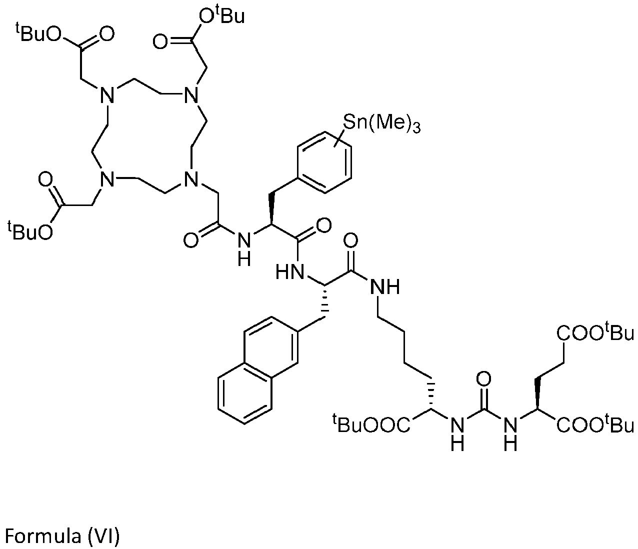 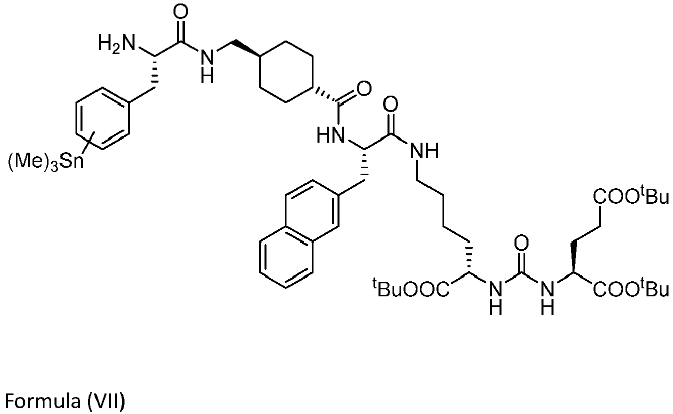 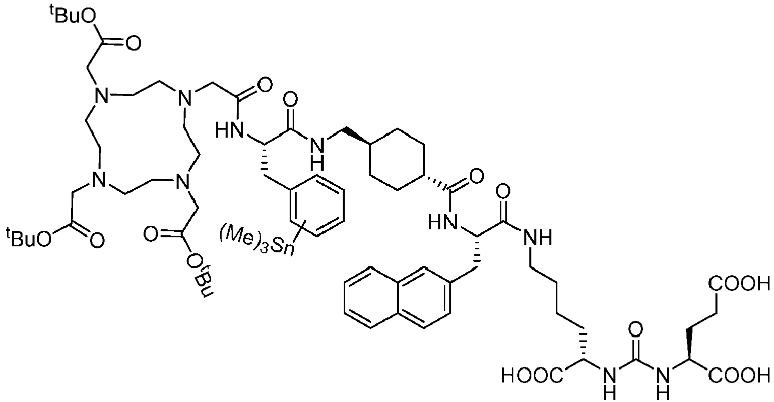 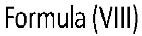 and

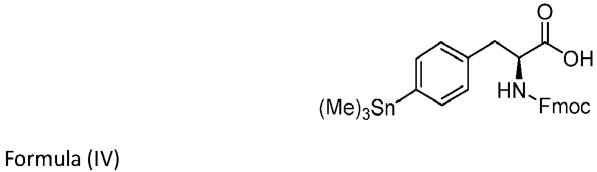

A preferred method comprises the following steps:
- Synthesis of a PSMA binding motif (BM)
- Coupling of linkers to BM, wherein one or more of the precursors of formula (II), (III), (V) and (VII) is provided
- Coupling of BM-linker to a chelator wherein one or more of the precursors of Formula (IV), (VI) and (VIII) is provided
- Labeling the BM-linker-chelator with a halogen radionuclide.

In a preferred embodiment, the PSMA binding motif is Lys-urea-Glu (LUG)

The PSMA targeting ligands of formula (I) such as the PSMA targeting ligands of formula (Ia), (Ib), (Ic), (Id), (Ie), (If), (Ig) and (Ih), can be used in radiotherapy, as imaging agents or as both i.e. as theranostic agents.

The pharmacokinetics of the PSMA targeting ligands of formula (I) that was tested outperform the already existing astatine radionuclide based probes.

In one aspect, the PSMA targeting ligands of formula (I) are for use in radiotherapy.

In another aspect, the PSMA targeting ligands of formula (I) are for use in the treatment of cancer, in particular prostate cancer.

In yet another aspect, the PSMA targeting ligands of formula (I) are for use as a theranostic agent.

In a further aspect of the invention is the use of PSMA targeting ligands of formula (I) as an imaging agent.

### Examples

### Example 1: GENERAL SYNTHETIC PROCDURES FOR THE SYNTHESIS OF DOTA-BEARING PSMA-TARGETING LIGANDS

### Glu-NCO⁸:

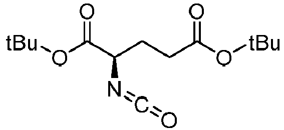

Firstly, di-*tert*-butyl *L*-glutamate hydrochloride (6.76 mmol) was suspended in a 1:2 mixture of dichloromethane (DCM) and saturated aqueous NaHCO₃ (72 mL). The mixture was cooled to 0 °C and then triphosgene (3.38 mmol) was added. The reaction mixture was vigorously stirred at 0 °C for 20 minutes, then warmed to room temperature, diluted with DCM and washed with brine (2x30 mL). The organic layers were collected, dried over Na₂SO₄ and concentrated *in vacuo,* affording the isocyanate Glu-NCO.

### PSMA binding motive⁸:

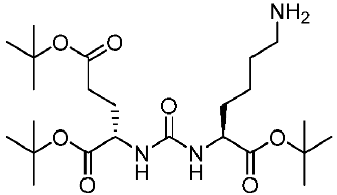

A solution of Glu-NCO (6.73 mmol) in DCM (16 mL) was added to a mixture of *tert*-butyl *N⁶*-((benzyloxy)carbonyl)-*L*-lysinate hydrochloride (7.40 mmol) and dry pyridine (7.40 mmol) in DCM (50 mL). The reaction was stirred at room temperature 18 hours. Afterwards, the reaction was diluted with 10 mL of DCM, washed with 0.1M HCI (5x15 mL) and washed with brine (2x15 mL). The organic layers were collected, dried over sodium sulphate and evaporated *in vacuo.* Finally, the obtained crude was purified by flash chromatography.

The Cbz-protected product (3.01 mmol) was weighted with Pd/C (10%) (0.31 mmol) catalyst, dissolved in MeOH (15 mL) and hydrogen gas was bubbled through the mixture overnight. The reaction mixture was filtered through a pad of diatomaceous earth and the solvent was evaporated *in vacuo,* yielding the PSMA binding motive.

### PSMA binding motive-linker:

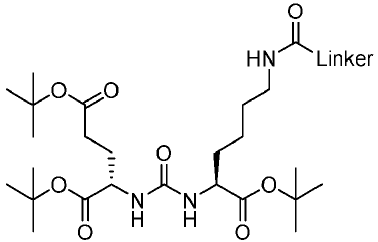

The subsequent syntheses of the PSMA-targeting peptidomimetics were carried out with the following general procedure:
*N*-Fmoc-amino acid (0.21 mmol) and diisopropylethylamine (DIPEA) (0.51 mmol) were dissolved in dry dimethylformamide (DMF), 1-[Bis(dimethylamino)methylene]-1*H*-1,2,3-triazolo[4,5-b]pyridinium 3-oxid hexafluorophosphate (HATU) (0.31 mmol) was added to the previous solution and stirred for 10 minutes. PSMA binding motive (0.21 mmol) was dissolved in dry DMF and dropwise added to the previous mixture for a total volume of 1 mL. The reaction mixture was stirred at room temperature for 4 to 24 hours depending on when completion was attained.
Thereafter, the *N*-terminus was deprotected by adding piperidine (50% relative to DMF) and stirring for 2 hours. Reaction mixture was poured over water and extracted with DCM. Organic layers were dried over Na₂SO₄ and volatiles removed *in vacuo.* The crude was purified by flash chromatography giving the desired free amine.

### PSMA binding motive-linker-chelator:

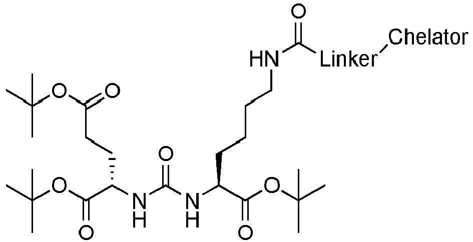

To a PSMA binding motive-linker construct solution (0.2 mmol) in either DCM or DMF (1 mL), trimethylamine (Et₃N) (0.31 mmol), DOTA-mono-NHS-tris(tBu-ester) (0.31 mmol) was added and stirred for 18 hours at room temperature. The subsequent crude mixture was purified by preparative HPLC.

### PSMA binding motive-linker-chelator radiolabelling:

A *tert*-butyl protected PSMA binding motive-linker-chelator construct was dissolved in a solution of Chloramine-T, methanol, ²¹¹At and acetic acid. The mixture was stirred and reacted during 30 minutes at room temperature. Afterwards, the mixture was dried by use of a nitrogen stream. The molecule was deprotected by addition of trifluoroacetic acid (TFA) and heating to 60 °C for 30 minutes. Once fully deprotected, the mixture was dried, redissolved in a 50:50 mixture of acetonitrile (MeCN)water and purified by preparative HPLC.

### Example 2: synthesis of PSMA-targeting radiopharmaceutical 1

Using the synthesis methods described in Example I, the following PSMA-targeting ligand was provided:
Synthesis of PSMA binding motif following the procedures previously described in Example I:

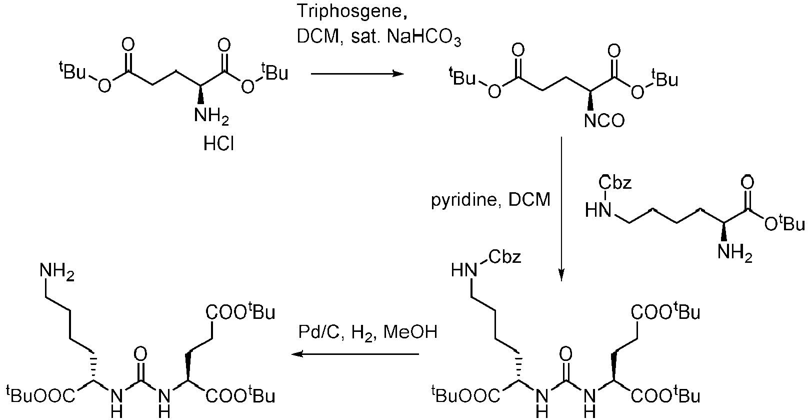

Coupling of linkers to the PSMA binding motive and labelling with ²¹¹At following the procedures previously described in Example I:

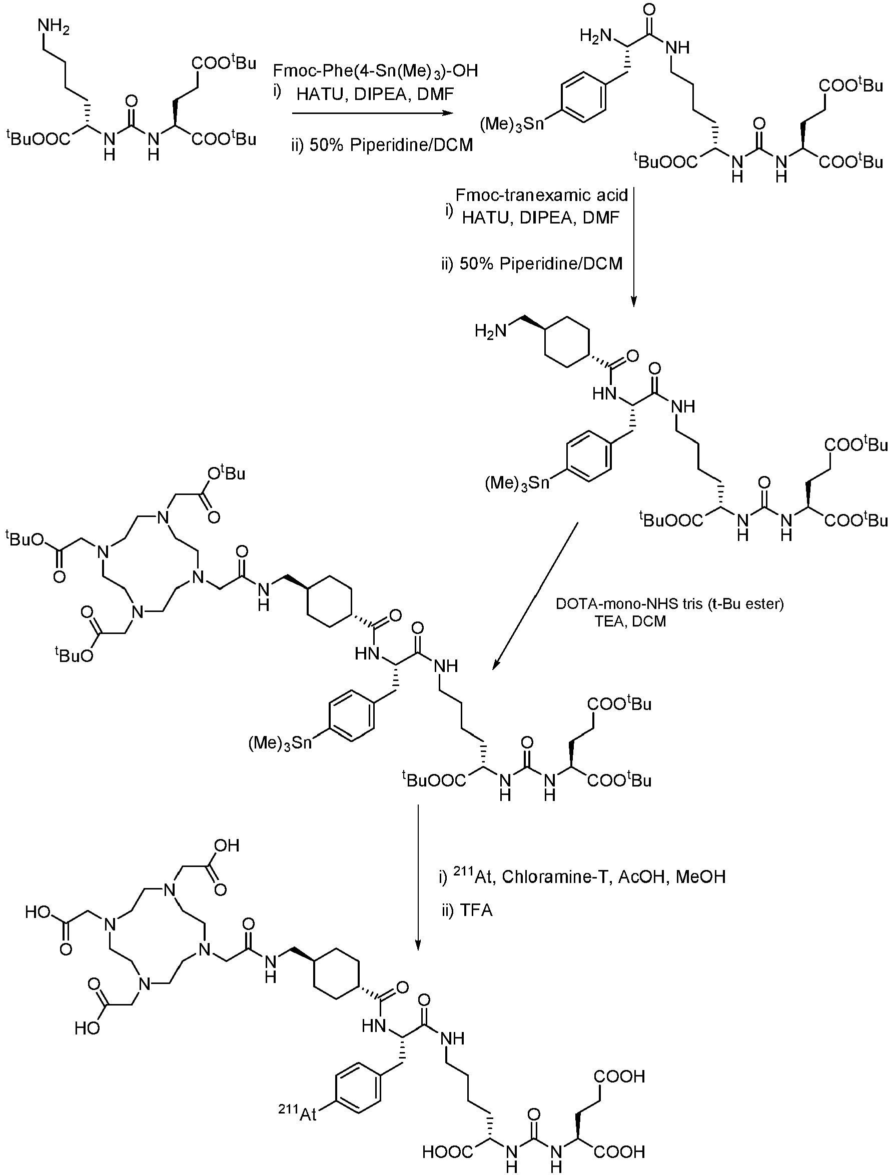

### Example 3: synthesis of PSMA-targeting radiopharmaceutical 2:

Using the synthesis methods described in Example I, the following PSMA-targeting ligand was provided:
Synthesis of PSMA binding motif followed by Coupling of linkers to LUG and labelling with ²¹¹At, following the procedures previously described in Example I:

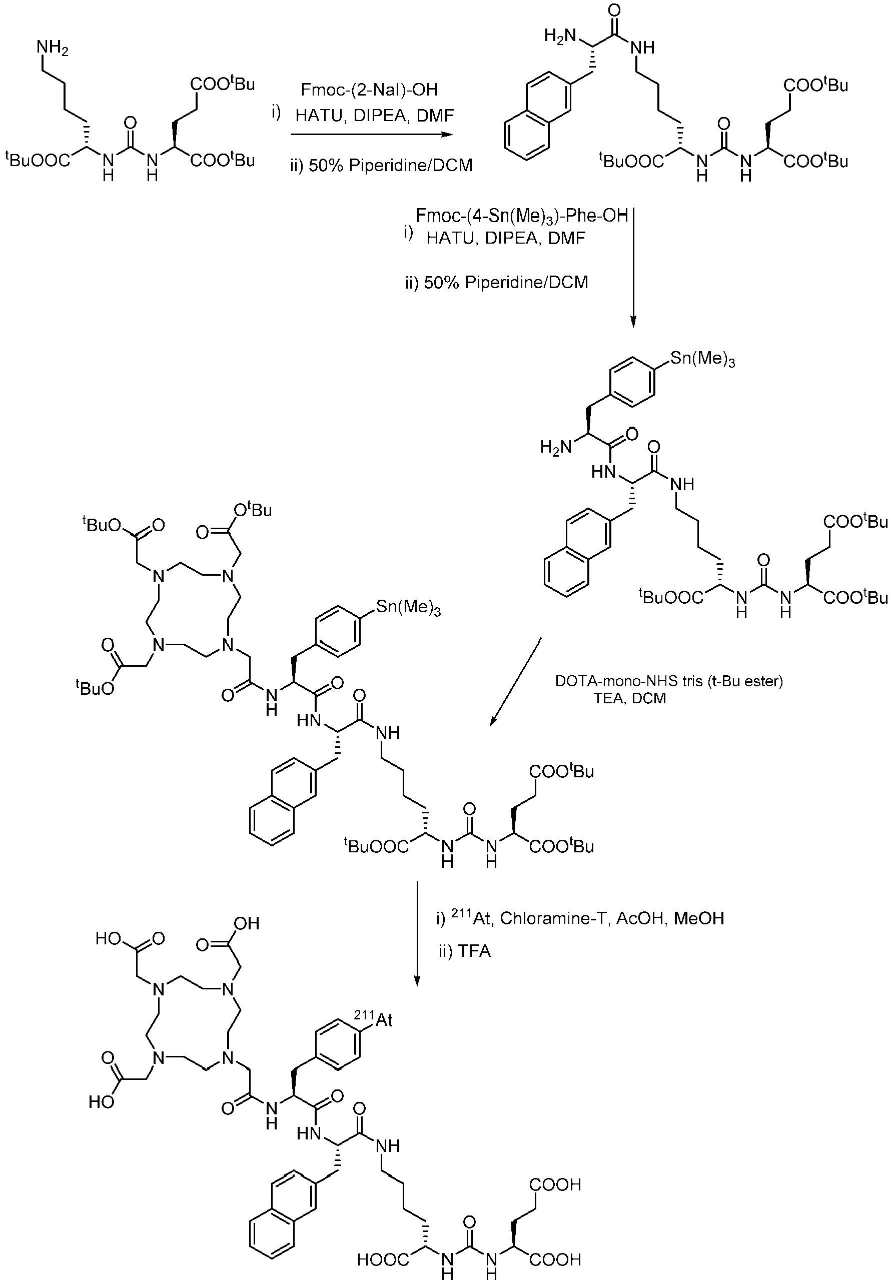

### Example 4: synthesis of PSMA-targeting radiopharmaceutical 3:

Using the synthesis methods described in Example I, the following PSMA-targeting ligand was provided:
Synthesis of the PSMA binding motif, followed by coupling of the linkers to the PSMA binding motive and labelling with ²¹¹At, following the procedures previously described in Example I:

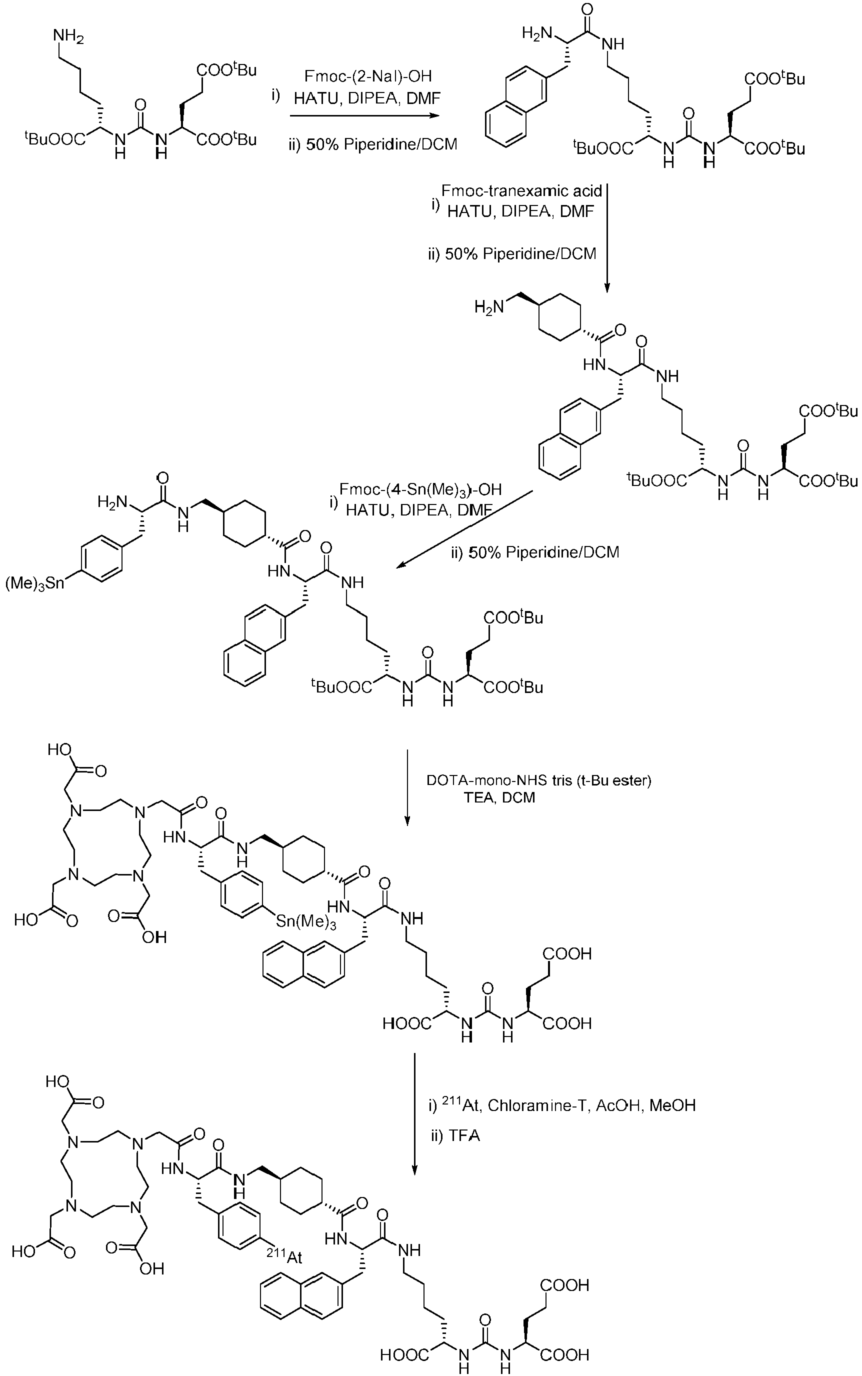

### Example 5: synthesis of (S)-2-((((9H-fluoren-9-yl)methoxy)carbonyl)amino)-3-(4-(trimethylstannyl)phenyl)propanoic acid, (Fmoc-(4-Sn(Me)₃)-OH):

Pd(PPh₃)₄ (0.19 mmol) and hexamethylditin (5.2 mmol) were added into a microwave vial equipped with a stirring bar. The vial was sealed and purged with nitrogen. Afterwards a solution of Fmoc-Phe(4-l)-OH (1 mmol) in 1,4-dioxane (5 mL) was added via syringe. The vial was allowed to react at 90 ºC in a microwave system for 2 hours. The reaction mixture was allowed to cool to room temperature and unsealed before being quenched with saturated aqueous KF solution (2 mL). The mixture was extracted with DCM, dried with sodium sulphate, filtered and solvents removed *in vacuo.* The crude product was purified via flash column chromatography.

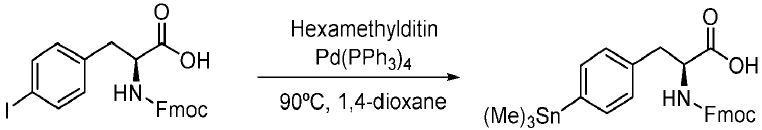

### Example 6: in vitro test of binding affinity and internalisation

The binding affinity of the PSMA targeting ligands provided in Examples 2, 3 and 4 towards PSMA is tested using PSMA+ LNCaP cells cultured in minimal essential medium, grown under controlled temperature and humidity. The cells are harvested and the affinity is determined in a competitive cell binding assay. LNCaP cells are incubated with a 0.2 nM solution of ⁶⁸Ga labelled ligand in the presence of different concentrations of non-radioactive ligands. After incubation at room temperature for 1 hour, the cell cultures are washed with ice-cold PBS buffer and the cell bound radioactivity is measured with a gamma counter. Affinity values (IC₅₀) are calculated fitting the obtained data by means of a nonlinear regression.

For determination of the internalisation, LNCaP cells are seeded in lysine coated plates 24 hours before the experiment. Cells are incubated at 37 ºC for 45 minutes with 32 nM of ⁶⁸Ga labelled radioligand (25 GBq/mol) in 250 uL minimal essential medium. The cells are washed with ice-cold PBS and with 50 mM glycine to remove surface bound radioactivity. After washing, the internalisation is determined by lysis of the cells using 0.3 M NaOH. Thereafter the radioactivity collected from the glycine and the hydroxide fraction is measured in a gamma counter.

The affinity and internalisation assays show that the ligands tested have good binding affinity and a sufficient cell internalisation, which are sufficient for theranostic applications.

### Example 7: in vivo evalution of the PSMA targeting radioligands

The most promising compounds (the ones with the lowest IC₅₀) are tested in human xenograft mice models. Two PSMA+ cell lines, LNCaP and PC-3M-LUC2 are used. LNCaP is a subcutaneous tumour cell line while PC-3M-LUC2.lu is metastatic disease with fluorescent cells that can be imaged by bioluminescence imaging (BLI).

For biodistribution studies, mice bearing LNCaP subcutaneous, 300-500 mm³ in volume, tumours are injected via the tail vein with 800 kBq of the radiohalogenated ligands formulated in PBS. Mice are sacrificed 0.5, 1, 2, 4, and 18 h after injection. Afterwards, tissues are isolated, weighted and the concentration of radionuclide is determined by gamma count in a scintillator. The ligands that show the most promising biodistribution profiles are tested for efficacy in a metastatic animal model. In this case the development of metastatic disease is monitored by BLI. Mice are injected with PC-3M-LUC2 cells in the left heart ventricle and then randomised into three groups. Group 1 receives the radiohalogenated compound, group 2 receives ¹⁷⁷Lu-PSMA-617 (current state-of-the-art) and group 3 receives vehicle injection (control). The metastatic burden is monitored by *in vivo* BLI over 4 weeks. The number of metastases, volume and overall survival are the values used to assess the efficacy of the radiohalogenated compounds.

Moreover the plasma half-life of the PSMA targeting ligands is measured during the course of treatment of the mice as well as the clearance and renal and salivary build-ups.

The results of the *in vivo* tests in mice show that the PSMA targeting ligands provided in Examples 2, 3 and 4 show good prospects for *in vivo* theranostic applications. The renal and salivary gland build-up is comparable to or less than the salivary gland build-up of the previously tested prior art PSMA targeting alpha radionuclide.

The half-life and clearance time shows that the pharmacokinetics of the presently disclosed compounds outperform the already existing alpha radionuclide based approaches.

### REFERENCES:

(1) Bray, F.; Ferlay, J.; Soerjomataram, I.; Siegel, R. L.; Torre, L. A.; Jemal, A. Global Cancer Statistics 2018: GLOBOCAN Estimates of Incidence and Mortality Worldwide for 36 Cancers in 185 Countries. CA. Cancer J. Clin. 2018, 68 (6), 394-424.
(2) Antonarakis, E. S.; Feng, Z.; Trock, B. J.; Humphreys, E. B.; Carducci, M. A.; Partin, A. W.; Walsh, P. C.; Eisenberger, M. A. The Natural History of Metastatic Progression in Men with Prostate-Specific Antigen Recurrence after Radical Prostatectomy: Long-Term Follow-Up. BJU Int. 2012, 109 (1), 32-39.
(3) De Bono, J. S.; Oudard, S.; Ozguroglu, M.; Hansen, S.; MacHiels, J. P.; Kocak, I.; Gravis, G.; Bodrogi, I.; MacKenzie, M. J.; Shen, L.; et al. Prednisone plus Cabazitaxel or Mitoxantrone for Metastatic Castration-Resistant Prostate Cancer Progressing after Docetaxel Treatment: A Randomised Open-Label Trial. Lancet 2010, 376 (9747), 1147-1154.
(4) Israeli, R. S.; Powell, C. T.; Corr, J. G.; Fair, W. R.; Heston, W. D. W. Expression of the Prostate-Specific Membrane Antigen. Cancer Res. 1994, 54 (7), 1807-1811.
(5) Benesová, M.; Schäfer, M.; Bauder-Wüst, U.; Afshar-Oromieh, A.; Kratochwil, C.; Mier, W.; Haberkorn, U.; Kopka, K.; Eder, M. Preclinical Evaluation of a Tailor-Made DOTA-Conjugated PSMA Inhibitor with Optimized Linker Moiety for Imaging and Endoradiotherapy of Prostate Cancer. J. Nucl. Med. 2015, 56 (6), 914-920.
(6) Pomper, M. G.; Mease, R. C.; Chen, Y.; Ray, S.; Zalutsky, M.; Vaidyanathan, G. PSMA TARGETED RADIOHALOGENATED UREAS FOR CANCER RADIOTHERAPY - WO2017070482A2, 2016.
(7) Pomper, M. G.; Mease, R. C.; Kumar, V.; Ray, S.; Zalutsky, M.; Vaidyanathan, G. PSMA TARGETED RADIOHALOGENATED UREA-POLYAMINOCARBOXYLATES FOR CANCER RADIOTHERAPY WO2019157037A1, 2019.
(8) Lowe, P. T.; Dall'Angelo, S.; Fleming, I. N.; Piras, M.; Zanda, M.; O'Hagan, D. Enzymatic Radiosynthesis of a 18 F-Glu-Ureido-Lys Ligand for the Prostate-Specific Membrane Antigen (PSMA). Org. Biomol. Chem. 2019, 17 (6), 1480-1486.

## Claims

1. PSMA targeting ligand of formula (I) 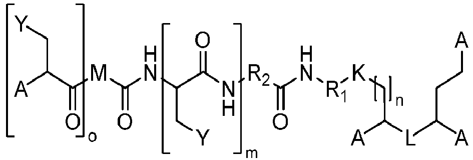 wherein:
A is independently carboxylic acid, sulphonic acid, phosponic acid, tetrazole or isoxazole;
L is selected from the group consisting of urea, thiourea, -NH-(C=O)-O-, -O-(C=O)-NH- or-CH2-(C=O)-CH2-;
K is selected from the group consisting of -(C=O)-NH-, -CH2-NH-(C=O)- or 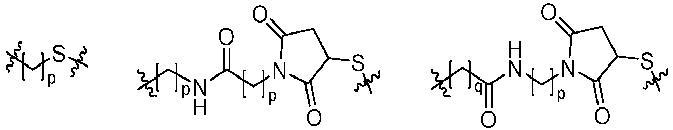 wherein
p is independently an integer selected from the group consisting of 1, 2, 3, 4, 5 and 6 ;
q is an integer selected from the group consisting of 0, 1, 2, 3, 4, 5 and 6 ;
Y is selected from the group consisting of: 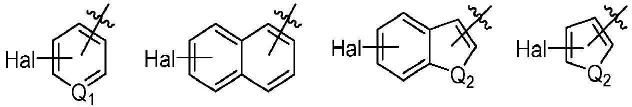 wherein
Q1 is -C-R3 or N, wherein R3 is H or C1-C5 alkyl;
Q2 is O, S orNH;
Hal is a radionuclide of the halogen group selected from the group consisting of a radioisotope of fluorine, a radioisotope of iodine, a radioisotope of bromine or a radioisotope of astatine;
M is a chelating agent, that can comprise a metal
n is an integer selected from the group consisting of 1, 2, 3, 4, 5 and 6 ;
m is an integer selected from the group consisting of 0 and 1;
o is an integer selected from the group consisting of 0 and 1;
R1 is -CH-CH2-Z or -CH-CH2-Y;
wherein Z is selected from the group consisting of: 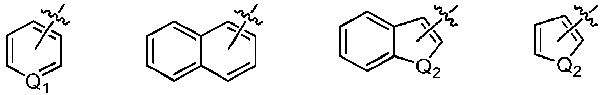 and Y is selected from the group consisting of: 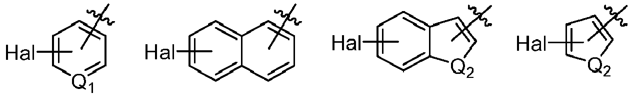 wherein
Q1 is -C-R3 or N, wherein R3 is H or C1-C5 alkyl;
Q2 is O, S orNH;
Hal is a radionuclide of the halogen group selected from the group consisting of a radioisotope of fluorine, a radioisotope of iodine, a radioisotope of bromine or a radioisotope of astatine;
R2 is -CH-CH2-Y or -CH2-X-;
wherein X is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 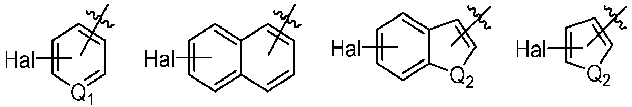 wherein
Q1 is -C-R3 or N, wherein R3 is H or C1-C5 alkyl;
Q2 is O, S orNH;
Hal is a radionuclide of the halogen group selected from the group consisting of a radioisotope of fluorine, a radioisotope of iodine, a radioisotope of bromine or a radioisotope of astatine;
and wherein formula (I) comprises at least one radioisotope selected from a radioisotope of fluorine, a radioisotope of iodine, a radioisotope of bromine or a radioisotope of astatine;
and pharmaceutically acceptable salts thereof.

2. PSMA targeting ligand according to claim 1, having the general formula (Ia): 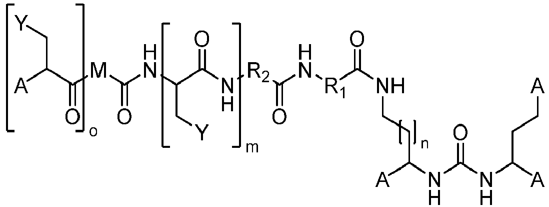 wherein:
A is independently carboxylic acid, sulphonic acid, phosponic acid, tetrazole or isoxazole;
n is an integer selected from the group consisting of 1, 2, 3 and 4 ;
m is an integer selected from the group consisting of 0 and 1;
o is an integer selected from the group consisting of 0 and 1;
Y is is selected from the group consisting of: 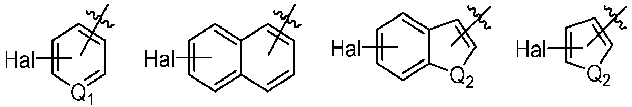 wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
M is a chelating agent, that can comprise a metal
R₁ is -CH-CH₂-Z or -CH-CH₂-Y;
wherein Z is selected from the group consisting of: 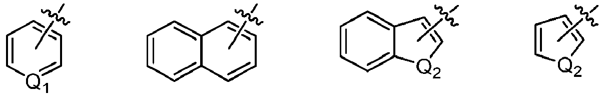 and Y is selected from the group consisting of: 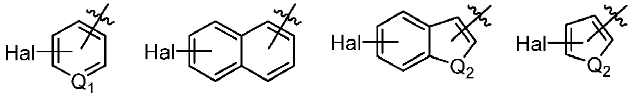 wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
R₂ is -CH-CH₂-Y or -CH₂-X-;
wherein X is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 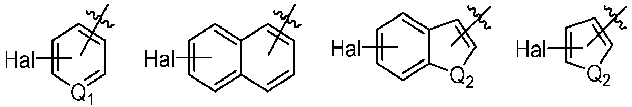 wherein
Q₁ is -C-R³ or N, wherein R³ is H or C₁-C₅ alkyl;
Q₂ is O, S or NH;
Hal is a radionuclide of the halogen group selected from the group consisting of a radioisotope of fluorine, a radioisotope of iodine, a radioisotope of bromine or a radioisotope of astatine;
and wherein formula (I) comprises at least one radioisotope selected from a radioisotope of fluorine, a radioisotope of iodine, a radioisotope of bromine or a radioisotope of astatine;
and pharmaceutically acceptable salts thereof.

3. PSMA targeting ligand according to claim 1 or 2, wherein M is selected from the group consisting of:
1,4,7,10-tetraazacyclododecane-N,N',N',N"-tetraacetic acid (DOTA), N,N'-bis(2-hydroxy-5-(carboxyethyl)benzyl)ethylenediamine N,N'-diacetic acid (HBED-CC), 14,7-triazacyclononane-1,4,7-triacetic acid (NOTA),
2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1-yl)pentanedioic acid DOTAGA),
4,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1(15),11,13-triene-3,6,9-triacetic acid (PCTA),
N'-(5-acetyl (hydroxy)aminopentyl-N-(5-(4-(5-aminopentyl)(hydroxy)amino-4-oxobutanoyl)amino)pentyl-N-hydroxysuccinamide (DFO),
diethylenetriaminepentaacetic acid (DTPA),
trans-cyclohexyl-diethylenetriaminepentaacetic acid (CHX-DTPA), 1-oxa-4,7,10-triazacyclododecane-4,7,10-triacetic acid (OXO-Do3A),
p-isothiocyanatobenzyl-DTPA (SCN-BZ-DTPA),
1-(p-isothiocyanatobenzyl)-3-methyl-DTPA (1B3M),
2-(p-isothiocyanatobenzyl)-4-methyl-DTPA (1M3B), and
1-(2)-methyl-4-isocyanatobenzyl-DTPA (MX-DTPA);
and pharmaceutically acceptable salts thereof.

4. PSMA targeting ligand according to any of the above claims, wherein M comprises a metal selected from the group consisting of Y, Lu, Tc, Zr, In, Sm, Re, Cu, Pb, Ac, Bi, Al, Ga, Ho and Sc.

5. PSMA targeting ligand according to any of the above claims, wherein R₁ is -CH-CH₂-Y and Hal is selected from the group consisting of ¹²⁵I, ¹²³I, ¹³¹I, ¹²⁴I, ²¹¹At, ⁷⁷Br and ^{80m}Br.

6. PSMA targeting ligand according to claim 1 or 2, selected from the group consisting of:

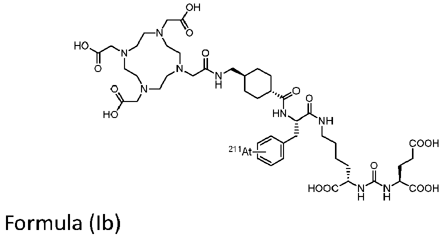

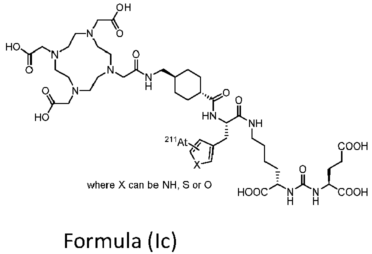

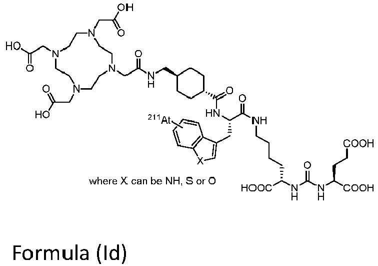

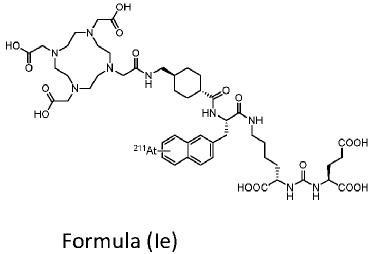

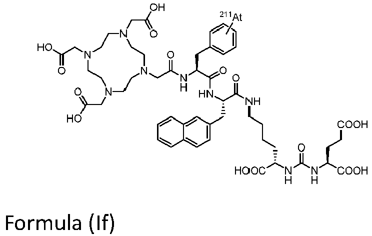

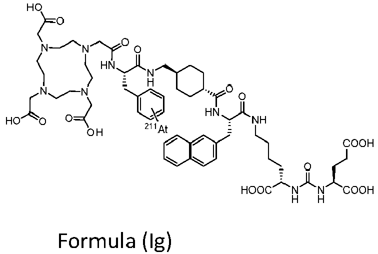

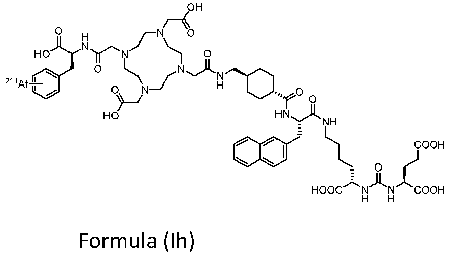

7. Precursor compound of the PSMA targeting ligand according to claim 1 or 2 selected from the group consisting of: 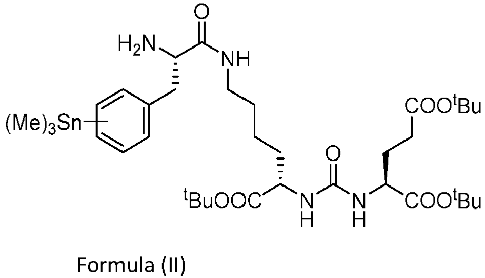 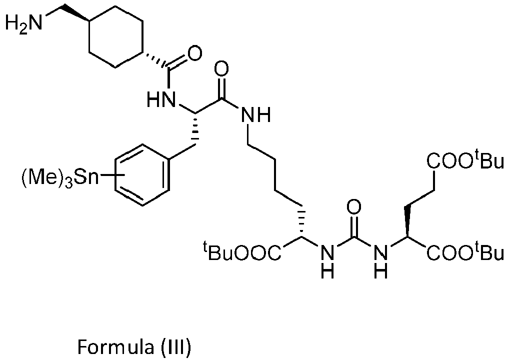 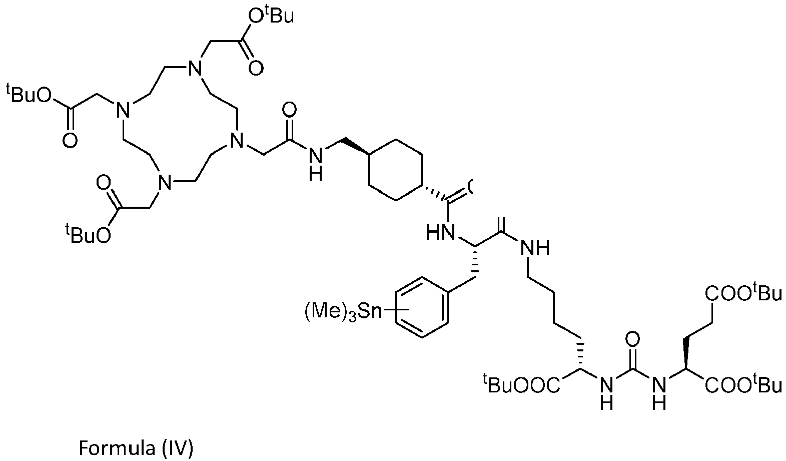 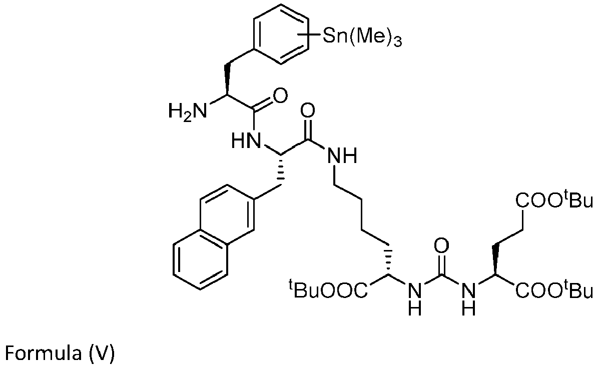 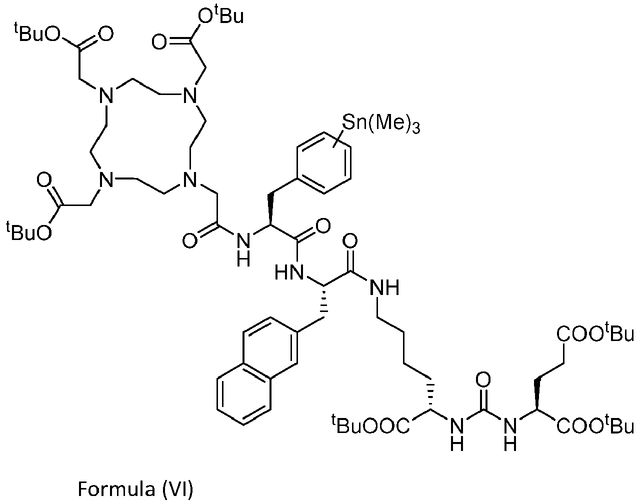 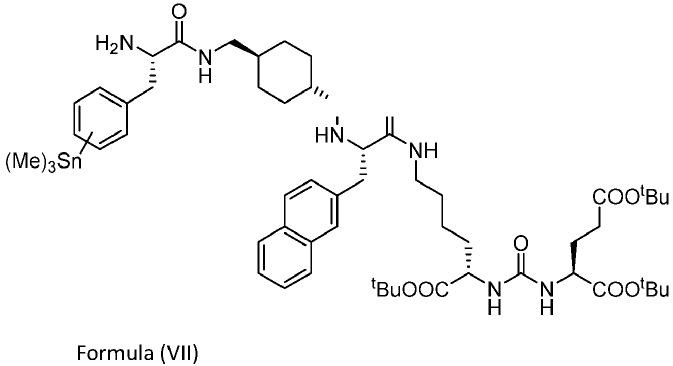 and

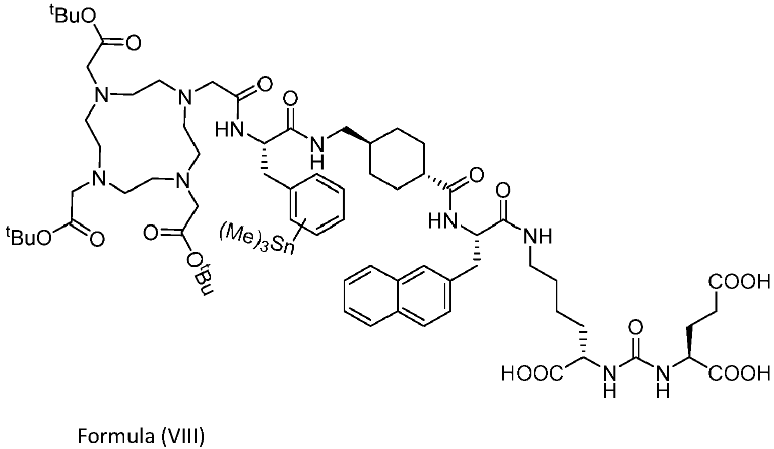

8. Method for providing the PSMA targeting ligand according to any of claims 1 to 6 comprising
- Synthesis of a PSMA binding motif
- Coupling of linkers to the PSMA binding motif, wherein one or more of the precursors of formula (II), (III), (V) and (VII) according to claim 7 is provided
- Coupling of the PSMA binding motif-linker to a chelator wherein one or more of the precursors of Formula (IV), (VI) and (VIII) according to claim 7 is provided
- Labeling the PSMA binding motif-linker-chelator with a halogen radionuclide.

9. Method according to claim 8, wherein the PSMA binding motif is Lys-urea-Glu.

10. Method according to claim 8 or 9, wherein the halogen radionuclide is selected from a radioisotope of fluorine, iodine, bromine or astatine.

11. Method according to claim 9 wherein the halogen radionuclide is selected from the group consisting of ¹²⁵I, ¹²³I, ¹³¹I, ¹²⁴I, ²¹¹At, ⁷⁷Br and ^{80m}Br.

12. PSMA targeting ligands of formula (l) according to any of claims 1 to 6 for use in radiotherapy.

13. PSMA targeting ligands of formula (I) according to any of claims 1 to 6 for use in the treatment of cancer, in particular prostate cancer.

14. PSMA targeting ligands of formula (I) according to any of claims 1 to 6 for use as a theranostic agent.

15. Use of PSMA targeting ligands of formula (I) according to any of claims 1 to 6 as an imaging agent.
